# EUROPÄISCHE PATENTANMELDUNG

(11) **EP 1 179 338 A1**
(43) Veröffentlichungstag der Anmeldung: **13.02.2002**
(21) Anmeldenummer: 01117245.9
(22) Anmeldetag: 17.07.2001
(51) Int. Cl.: A61K 7/48, A61K 7/06

(54) **Kosmetische und dermatologische Zubereitung mit einem Gehalt an Cyclodextrinen zur Beseitigung von Sebum**

(30) Priorität: 10.08.2000 DE 10039063
(71) Anmelder: Beiersdorf AG, 20245 Hamburg (DE)
(72) Erfinder: Max, Heiner, Dr., 22529 Hamburg (DE); Nielsen, Jens, 24558 Henstedt-Ulzburg (DE); Raschke, Thomas, Dr., 25421 Pinneberg (DE)

(57) **Zusammenfassung**

Verwendung von Cyclodextrinen zur Herstellung von Zubereitungen zur Verminderung der Produktion von Sebum bzw. die Verwendung von Cyclodextrinen zur Herstellung von Zubereitungen zur Beseitigung von Sebum.

## Beschreibung

Die vorliegende Erfindung betrifft die Verwendung von topischen Zubereitungen zur Entfernung von Sebum von der Haut, insbesondere zur Beseitigung und Klärung von Comedonen, zur Vermeidung von Comedonenbildung, zur Prophylaxe und Behandlung leichter Formen der Akne sowie zur Prophylaxe und Bekämpfung der Seborrhöe.

Talgdrüsenüberproduktion und die möglichen sich daraus entwickelnden Hautstörungen wie Talgretention, Ausbildung von Comedonen im Gebiet der Talgdrüsen, also im Gesicht (vor allem Stirn, Nase und Kinn) und auf dem oberen Rücken und in der Folge die verschiedenen Formen der Akne, sind ein häufig anzutreffendes Hautproblem, das bisher nicht in zufriedenstellendem Masse gelöst worden ist.

Talg ist das Sekret der Talgdrüse. Talgdrüsen sind Ausbuchtungen des Follikelepithels und damit Bestandteile des Follikels, mit dem sie eine funktionelle Einheit bilden. Sie sind holokrine Drüsen, das bedeutet, die ganze Drüsenzelle wird zum größten Teil in das Sekret Talg umgewandelt. Die Zellen der Talgdrüse, die von unten her ständig emeuert werden, verfetten und zerfallen, und der so gebildete Hauttalg wird durch die Follikelmündungen auf die Hautoberflache entleert.

Der Hauttalg besteht nach G. Leonardy (J.Ft. Jellinek Kosmetologie, Zweck und Aufbau kosmetischer Präparate, Dr. Alfred Hüthig-Verlag, Heidelberg - Mainz - Basel, dritter völlig überarbeiteter und wesentlich erweiterte Auflage 1976, Seiten 26 bis 29) aus Mono-, Di- und Triglyceriden (C₁₀-C₁₈), Wachsen (C₁₆-C₂₆), Wachsestern (C₂₈-C₃₈), normalen gesättigten Fettsäuren (C₁₀-C₁₈), verzweigtkettigen gesättigten Fettsäuren (C₁₁-C₁₈), mehrfach verzweigtkettigen gesättigten Fettsäuren (C₁₃-C₁₈), einfach ungesättigten Fettsäuren (C₁₁-C₁₈), mehrfach ungesättigten Fettsäuren (C₁₃, C₁₅-C₁₇), Sterolen (Cholesterin, 7-Dehydrocholesterin, 7-Hydroxycholesterin), verzweigten und unverzweigten Kohlenwasserstoffen (C₃₀-C₄₀), Squalen und Phospholipiden.

Zusammen mit dem wässrigen Sekret der ekkrinen Schweißdrüsen bilden die Lipide der Talgdrüse den sogenannten Hydro-Lipid-Film der Haut. Dieser Oberflächenfilm ist eine Emulsion, die eine Wasser-in-Öl oder eine Öl-in-Wasser-Emulsion sein kann. Sie hat die Funktion, die Hautoberfläche geschmeidig zu halten und den Wassergehalt der tieferliegenden Hautschichten zu regeln. Bei guter Hydratation des Sebums beträgt der Wassergehalt mindestens 10 bis 20 Gew.-% und das Sebum ist hydrophil. Ist das hydrophillipophile Gleichgewicht des Oberflächenfilmes nicht mehr gegeben und der Wassergehalt sinkt, so verändert sich das Sebum und wird hydrophob. Der Talgabfluss aus Talgdrüse und Follikel wird behindert. Es kommt zu einer Stauung des Hauttalgs in den Follikelmündungen, die dann in der Folge zu Comedonen und Entzündungen der Follikel führen kann.

Die Veränderung des Hauttalgs und das Einleiten der Comedonenbildung kann verschiedene Ursachen haben. Zum Beispiel: äußere Einflüsse, wie falsche Reinigungsgewohnheiten und falsche Pflege, comedogene Substanzen in Kosmetika, Witterungseinflüsse, alkalische Seifen, scharfe Detergentien. Eine vermehrte Talgdrüsensekretion und die Ausbildung von Comedonen kann sich auch durch genetische Faktoren und hormonelle Einflüsse entwickeln. Auch hier können Comedonen, Entzündungen, Prä-Akne und Akne mit ihren Nachwirkungen die Folge sein.

Die Häufigkeit der Hautschäden durch eine gestörte Talgdrüsenfunktion und Krankheiten der Talgdrüsen steigen immer mehr, und die Rückführung/Vermeidung der Comedonenbildung ist somit ein vordringliches Anliegen. Doch bisherige Versuche, die Comedonenbildung als ein ursächliches Problem zu lösen, haben zu wenig befriedigenden Ergebnissen geführt.

Neben dem manuellen Entfemen der Comedonen durch Ausdrücken sind zahlreiche Reinigungsmethoden bekannt, mit denen versucht wird, Comedonen zu entfernen und die Comedonenbildung nachhaltig zu verhindem. Hierzu gehören spezielle Seifen, hautabschälende Kompositionen und dergleichen. Erweichende und adstringierende Mittel finden ebenso Anwendung. Darüber hinaus wird versucht, durch Zusatz von austrocknenden, keratolytischen, antiseborrhoischen und antibakteriellen Wirkstoffen in kosmetischen und pharmazeutischen Zubereitungen die Neigung zur Akne zu reduzieren, ohne daß sich Irritationen der Haut oder ein Austrocknen der Haut einstellen.

Die Hautreinigung entfettet allerdings die Haut und entzieht ihr Feuchtigkeit. Zusätzlich haben Seifen den Nachteil, daß die sich bei Verwendung der Seifen in hartem Wasser bildenden wasserunlöslichen Calcium- und Magnesiumsalze der höheren Fettsäuren auf der Haut schleimige Niederschläge bilden. Diese Niederschläge verbleiben bei schlechter Abspülbarkeit länger auf der Haut, verstopfen die Follikelmündungen und können zu einer Ausbildung von Comedonen führen. Daher werden zur Hautreinigung vorwiegend Syndets (d.h., Tenside ohne Seifencharakter) in Form von Waschcrèmes oder Waschlotionen eingesetzt. Diese Syndets bilden zwar keine Kalkseifen, aber die Behandlung mit stark oberflächenaktiv wirkenden Agentien hat auf die Haut eine stärker entfettende und austrocknende Wirkung als Seife. Je häufiger seifen- und tensidhaltige Produkte auf der Haut angewandt werden, desto deutlicher treten deren nachteilige Wirkungen, nämlich Entfettung und Austrocknung der Haut durch Zerstörung des Hydro-Lipid-Filmes in den Vordergrund. Fast immer führt die Reduzierung der Comedonen zur Absenkung des Wassergehaltes in den oberen Hautschichten und zu einer festen Konkrementbildung in den Talgdrüsen, die wiederum Entzündungen induzieren kann. Die Absenkung des Feuchtigkeitsgehaltes der Haut ist aber für eine pflegende Entfernung der Comedonen kontraproduktiv.

Seborrhöe ist eine gesteigerte Funktion der Talgdrüsen durch Veranlagung. Sowohl Kopfhaut als auch Gesichtshaut erscheinen fettig. Die Zusammensetzung des seborrhoischen Sebums ist gegenüber dem normalen Sebum verändert. Man unterscheidet 3 Entwicklungsstufen der Seborrhoe:
1. Einfache Seborrhöe Leichte Fälle, fettig nach 8 Tagen.
2. Ölige Seborrhöe bereits nach 2-3 Tagen fettig.
3. Irreversible Form nicht mehr umkehrbar. Die Seborrhöe, bei der das Haar bereits nach einem Tag wie in Fett gebadet aussieht.

Die übermäßige Absonderung der Talgdrüsen kann unter anderem durch androgenetische Störungen ausgelöst werden und hat einen ästhetischen Nachteil auf das Gesamtbild der Haare. Diese Störung kann auch die Ursache für auftretenden Haarausfall sein. Vorläufer ist jeweils der seborrhoische Zustand der Kopfhaut. Vegetative Störungen sowie unsachgemäße Pflege können das Hautbild und auch den Haarzustand noch verschlechtem. Auch bei Seborrhöe kann das Haar selbst durch Störungen im Keratinaufbau trocken sein. Trockenes, angegriffenes Haar wird häufig hervorgerufen durch äußere Beanspruchung wie z. B. Sonne oder chemischen Behandlungen. Zu heißes Fönen oder nicht richtige Pflege von angegriffenem Haar können zu Schädigungen führen.

Die Ursachen für fettiges Haar liegen im Körper des Menschen und sind hormonell bedingt. Jedes Haar besitzt eine eigene Talgdrüse, die Fett (Sebum, Hauttalg) produziert. Die Sebum-Produktion wird hormonell gesteuert, und es kann je nach Hormonempfindlichkeit der Talgdrüse zu einer Über- oder Unterproduktion kommen. Der Talg selber hat die Funktion die Kopfhaut geschmeidig zu halten. Er gelangt aus der Talgdrüse auf die Kopfhaut und erst später den Haaransatz. Dort wird er normalerweise vom Haarschaft aufgenommen und bleibt unsichtbar. Bei einer Talgüberproduktion ist der Haarschaft nicht mehr in der Lage diesen aufzunehmen. Er wird als Fettfilm am Haar sichtbar. Die Folge ist strähniges, fettig glänzendes Haar.

Dadurch daß die Talgdrüsenproduktion vom Hormonhaushalt abhängig ist, läßt sich das Problem fettigen Haares nicht grundsätzlich lösen, denn die Talgdrüsen produzieren stetig Fett. Konsequente Pflege und hochwertige Pflegeserien sind immer noch die beste Methode zur Bekämpfung fettigen Haares.

Fettiges Haar hat sehr lästige Auswirkungen. Die Haare werden schon kurze Zeit nach dem Waschen wieder strähnig und die Frisur hält nicht.

Entgegen landläufiger Meinung ist es nur ein Gerücht, daß durch zu häufiges Waschen das Haar noch schneller fettig wird. Milde Shampoos gegen fettiges Haar sorgen dafür, daß übermäßiges Fett entfernt wird. Haar und Kopfhaut werden mit ausreichend Feuchtigkeit versorgt und gleichen die Überproduktion der Talgdrüsen aus.

Fettiges Haar und Schuppenbildung gehören zu den häufigsten Haarproblemen. Diese Anomalien sind auf eine Störung der Talgdrüsentätigkeit zurückzuführen. Bei einer Überfunktion der Talgdrüsen sprechen wir von einer Seborrhoe. Dabei sind zwei Formen zu unterscheiden: die ölige Form (Seborrhöe oleosa) und die trockene Form (Seborrhöe sicca).

### Die Seborrhoea oleosa :

Hier liegt eine Überfunktion der Talgdrüsen vor, bei der die Talgdrüsen zu viel und zu öligen Talg erzeugen. Die Haut zeigt deshalb einen fettigen Glanz, und die Haare sind schon 2 bis 3 Tage nach der Wäsche wieder bis in die Spitzen fettig und strähnig.

### Die Seborrhoea sicca :

Sie ist ebenfalls auf eine Überfunktion der Talgdrüsen zurückzuführen, aber der Hauttalg ist trockener, er hat eine festere Konsistenz. Mit den Schüppchen der Oberhaut bildet er große, leicht zerreibbare Talgschuppen. Die Kopfhaut zeigt einen wachsartigen Glanz, das Haar fettet nur am Ansatz nach, die Längen und besonders die Spitzen sind trocken und sogar spröde.

Zur Behandlung der Seborrhöe gehört zunächst mal die regelmäßige und gründliche Kopfwäsche mit Spezialshampoos, die so oft durchgeführt werden kann, wie es nötig erscheint. Die Wäsche sollte mit einer Massage im Bindegewebe verbunden sein, weil dadurch dir Talgdrüsen stärker entleert werden, was die Nachfettung verzögert.

Der vorliegenden Erfindung liegt die Aufgabe zugrunde, eine Zubereitung zur Verfügung zu stellen, die die Nachteile der bekannten und bisher verwendeten Mittel nicht aufweist, die gleichzeitig talglösend, fettregulierend und pflegend wirkt, die bereits vorhandenen Comedonen ohne Irritationen entfernt, die Entfettung und Austrocknung der Haut wirksam verhindert, die Comedonenbildung und die Entstehung von Akne Kosmetika verhindert, sowie schon bestehende Akne bessert und die gleichwertig die Haut pflegt.

Diese Aufgabe wird erfindungsgemäß durch die Verwendung von Cyclodextrinen zur Herstellung von Zubereitungen zur Verminderung der Produktion von Sebum bzw. die Verwendung von Cyclodextrinen zur Herstellung von Zubereitungen zur Beseitigung von Sebum gelöst.

Überraschenderweise wird außerdem bei Verwendung der Zubereitung die comedogene Wirkung der in der Zubereitung verwendeten Rohstoffe aufgehoben und damit die Bildung von Comedonen und entsprechend die Entstehung von Akne verhindert.

Es hat sich ferner herausgestellt, daß die erfindungsgemäß verwendeten Wirkstoffe die Bildung von seborrhoischen Erscheinungen, insbesondere fettiges Haar, aber auch Kopfschuppen, verhindern sowie bereits vorhandene seborrhoische Erscheinungen, insbesondere fettiges Haar, aber auch Kopfschuppen, zu beseitigen.

Darüber hinaus gewährleistet die vorliegende Erfindung eine sehr gute Hautpflege, verbunden mit einer effizienten Entfernung überschüssigen Hauttalgs.

Erfindungsgemäß sind somit auch ein Verfahren zur Bekämpfung von unreiner Haut, Akne, oder seborrhoischen Erscheinungen, insbesondere fettigem Haar und/oder Kopfschuppen, dadurch gekennzeichnet, daß die erfindungsgemäß verwendeten Wirkstoffe in einem geeigneten kosmetischen oder dermatologischen Träger, mit dem von gesteigerte Sebumproduktion betroffenen Bereich in Kontakt gebracht werden.

Der Stand der Technik lieferte nicht den geringsten Hinweis auf die erfindungsgemäße Verwendung als antiseborrhoisches Wirkprinzip.

Eine weitere bevorzugte Ausführungsform der vorliegenden Erfindung sind mithin gegen Kopfschuppen anzuwendende Formulierungen, beispielsweise Antischuppenshampoos.

Cyclodextrine (Cycloamylosen, Cycloglucane) sind in kosmetischen und pharmazeutischen Zubereitungen an sich bekannt. Oftmals werden diese Substanzen zur "molekularen Verkapselung" verwendet, also als schützende Umhüllung empfindlicher Moleküle. Es handelt sich dabei um 6, 7, 8 oder noch mehr α-1,4-verknüpfte Glucoseeinheiten, wobei die Cyclohexaamylose (α-Cyclodextrin) sich durch die Struktur 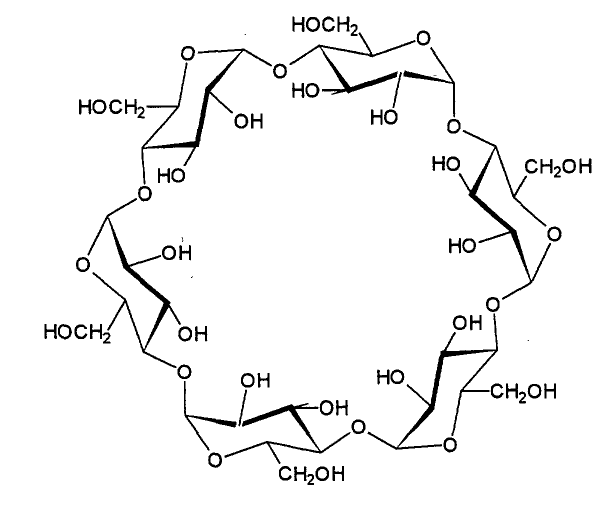 auszeichnet. Die Cycloheptaamylose (β-Cyclodextrin) zeichnet sich durch die Struktur 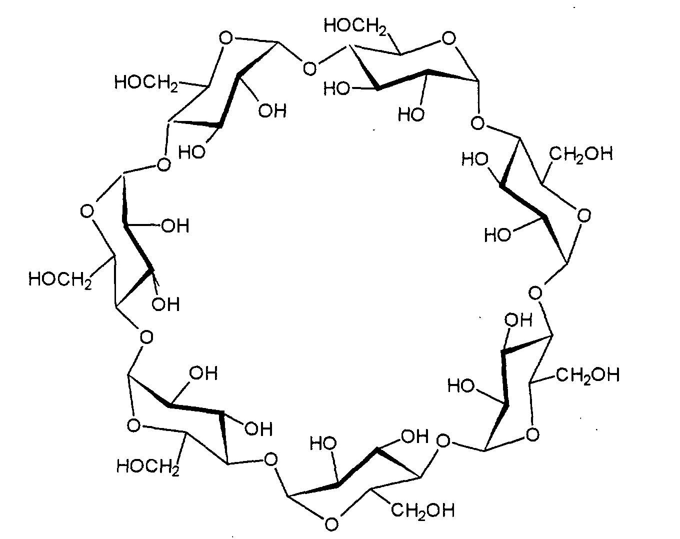 aus. Die Cyclooctaamylose (γ-Cyclodextrin) zeichnet sich durch die Struktur 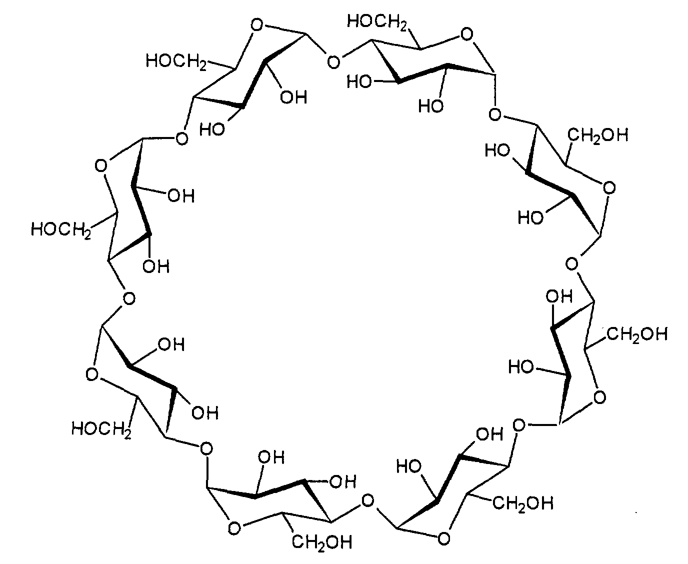 aus. Die Cycloenneaamylose (δ-Cyclodextrin) zeichnet sich durch die Struktur 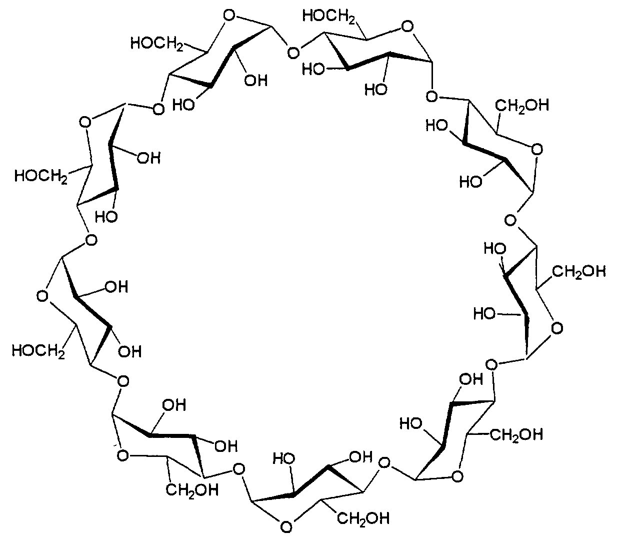 aus.

Weiterhin können im Rahmen dieses Patentes polar- und unpolar- substituierte Cyclodextrine eingesetzte werden. Hierzu gehören vorzugsweise aber nicht ausschließlich Methyl-, Ethyl- sowie Hydroxypropyl-Cyclodextrin.

Erfindungsgemäß bevorzugt ist das γ-Cyclodextrin, bzw. Gemische aus Cyclodextrinen, welche mindestens zu 30 Gew.-%, bezogen auf das Gesamtgewicht des Cyclodextringemisches an γ-Cyclodextrin enthalten.

Erfindungsgemäß werden das oder die Cyclodextrine bevorzugt in kosmetischen oder dermatologischen Zusammensetzungen eingesetzt einem Gehalt von 0,0005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an den erfindungsgemäß verwendeten Cyclodextrinen, ganz besonders vorteilhaft 0,5 - 3,0 Gew.-%, jeweils bezogen auf das Gesamtgewicht der Zusammensetzung.

Die erfindungsgemäß verwendeten Wirkstoffe lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen und dergleichen.

Es ist auch möglich und gegebenenfalls vorteilhaft, die erfindungsgemäß verwendeten Wirkstoffe mit anderen Wirkstoffen zu kombinieren, beispielsweise mit anderen antimikrobiell, antimycotisch bzw. antiviral wirksamen Stoffen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,5 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallussäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Auch die Verwendung zusätzlicher antimikrobiell, insbesondere antibakteriell wirksamer Substanzen ist erfindungsgemäß gegebenenfalls besonders vorteilhaft.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Crème, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Es ist beispielsweise vorteilhaft im Sinne der vorliegenden Erfindungen, einen Gehalt an UV-Schutzsubstanzen zu verwenden.

Vorteilhaft können daher erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure-(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bomylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäß verwendeten kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofem die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders bevorzugt wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Ganz besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase femer einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -mono-butylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen.

Die folgenden Beispiele sollen die Verkörperungen der vorliegenden Erfindungen verdeutlichen. Die Angaben beziehen sich stets auf Gewichts-%, sofern nicht andere Angaben gemacht werden.

| **Beispiel 1 (O/W-Creme):** | |
|---|---|
| | Gew.-% |
| Glycerylstearatcitrat | 2,00 |
| Stearylalkohol | 5,00 |
| Caprylisäure/Caprinsäuretriglyceride | 4,00 |
| Octyldodecanol | 4,00 |
| Glycerin | 3,00 |
| Carbomer | 0,10 |
| α-Cyclodextrin | 1,00 |
| Natriumhydroxid | q.s. |
| Konservierung | q.s. |
| Parfum | q.s. |
| Wasser, demineralisiert | ad 100,00 |
| pH-Wert eingestellt auf 6,0 | |

| **Beispiel 2 (O/W-Creme):** | |
|---|---|
| | Gew.-% |
| Glycerylstearatcitrat | 3,00 |
| Cetylstearylalkohol | 3,00 |
| Paraffin Oil | 2,00 |
| Caprylsäure-/Caprinsäuretriglyceride | 4,00 |
| Dicaprylylether | 3,00 |
| Xanthangummi | 0,10 |
| Citronensäure | 0,10 |
| Natriumcitrat | 0,20 |
| β-Cyclodextrin | 5,00 |
| Glycerin | 3,00 |
| Konservierung | q.s. |
| Parfum | q.s. |
| Wasser | ad 100,00 |
| pH-Wert eingestellt auf 5,5 | |

| **Beispiel 3 (O/W-Creme):** | |
|---|---|
| | Gew.-% |
| Glycerylstearat SE | 4,00 |
| PEG-40 Stearate | 1,00 |
| Cetylalkohol | 3,00 |
| Caprylsäure-/Caprinsäuretriglyceride | 5,00 |
| Paraffin Oil | 5,00 |
| Glycerin | 3,00 |
| Carbomer | 0,10 |
| γ-Cyclodextrin | 2,00 |
| Natriumhydroxid | q.s. |
| Konservierung | q.s. |
| Parfum | q.s. |
| Wasser, demineralisiert | ad 100,00 |
| pH-Wert eingestellt auf 5,0 | |

| **Beispiel 4 (O/W-Creme):** | |
|---|---|
| | Gew.-% |
| Glycerylstearat SE | 3,00 |
| Stearinsäure | 1,00 |
| Cetylalkohol | 2,00 |
| Dicaprylylether | 4,00 |
| Caprylsäure-/Caprinsäuretriglyceride | 3,00 |
| Paraffin Oil | 2,00 |
| Glycerin | 3,00 |
| Butylenglycol | 3,00 |
| Carbomer | 0,10 |
| γ-Cyclodextrin | 1,00 |
| β-Cyclodextrin | 5,00 |
| Natriumhydroxid | q.s. |
| Konservierung | q.s. |
| Parfum | q.s. |
| Wasser, demineralisiert | ad 100,00 |
| pH-Wert eingestellt auf 7,0 | |

| **Beispiel 5 (O/W-Lotion):** | |
|---|---|
| | Gew.-% |
| Glycerylstearat, Ceteth-20 | 1,00 |
| Sorbitan Stearate | 1,00 |
| Stearylalkohol | 1,00 |
| Caprylsäure-/Caprinsäuretriglyceride | 2,00 |
| Paraffinöl | 4,00 |
| Glycerin | 3,00 |
| Carbomer | 0,10 |
| β-Cyclodextrin | 5,00 |
| γ-Cyclodextrin | 2,00 |
| Natriumhydroxid | q.s. |
| Konservierung | q.s. |
| Parfum | q.s. |
| Wasser, demineralisiert | ad 100,00 |
| pH-Wert eingestellt auf 5,5 | |

| **Beispiel 6 (W/O/W-Creme):** | |
|---|---|
| | Gew.-% |
| Glycerylstearat SE | 3,00 |
| PEG-100-stearat | 0,75 |
| Behenylalkohol | 2,00 |
| Caprylsäure-/Caprinsäuretriglyceride | 8,0 |
| Octyldodecanol | 5,00 |
| C₁₂₋₁₅ Alkylbenzoate | 3,00 |
| Panthenol | 3,00 |
| MgSO₄ | 0,80 |
| β-Cyclodextrin | 5,00 |
| Konservierung | q.s. |
| Parfum | q.s. |
| Wasser, demineralisiert | ad 100,00 |
| pH-Wert eingestellt auf 6,0 | |

| **Beispiel 7 (Hydrodispersionsgel):** | |
|---|---|
| | Gew.-% |
| Carbomer | 0,40 |
| Xanthangummi | 0,20 |
| Cetylstearylalkohol | 2,00 |
| C₁₂₋₁₅ Alkylbenzoate | 5,00 |
| Caprylsäure-/Caprinsäuretriglyceride | 3,00 |
| Glycerin | 3,00 |
| α-Cyclodextrin | 0,20 |
| Natriumhydroxid | q.s. |
| Konservierung | q.s. |
| Parfum | q.s. |
| Wasser, demineralisiert | ad 100,00 |
| pH-Wert eingestellt auf 5,5 | |

| **Beispiel 8 (W/O-Creme):** | |
|---|---|
| | Gew.-% |
| PEG-7 Hydriertes Ricinusöl | 4,00 |
| Wollwachsalkohol | 1,50 |
| Bienenwachs | 3,00 |
| Paraffinöl | 10,00 |
| Caprylsäure-/Caprinsäuretriglyceride | 5,00 |
| Vaseline | 7,00 |
| Glycerin | 3,00 |
| MgSO₄ | 0,70 |
| β-Cyclodextrin | 1,00 |
| Konservierung | q.s. |
| Parfum | q.s. |
| Wasser, demineralisiert | ad 100,00 |

## Patentansprüche

1. Verwendung von Cyclodextrinen zur Herstellung von Zubereitungen zur Verminderung der Produktion von Sebum bzw. die Verwendung von Cyclodextrinen zur Herstellung von Zubereitungen zur Beseitigung von Sebum.

2. Verwendung nach Anspruch 1 zur Verhinderung der Bildung von Comedonen und/oder Akne.

3. Verwendung nach Anspruch 1 zur Verhinderung oder Beseitigung von seborrhoischen Erscheinungen, insbesondere fettiges Haar, aber auch Kopfschuppen.

4. Verwendung nach Anspruch 1, **dadurch gekennzeichnet, daß** das oder die Cyclodextrine gewählt werden aus der Gruppe α-Cyclodextrin, β-Cyclodextrin, γ-Cyclodextrin, δ-Cyclodextrin, sowie aus der Gruppe der Gemische aus Cyclodextrinen, welche mindestens zu 30 Gew.-%, bezogen auf das Gesamtgewicht des Cyclodextringemisches an γ-Cyclodextrin enthalten.

5. Kosmetisches Verfahren zur Bekämpfung von unreiner Haut, Akne, oder seborrhoischen Erscheinungen, insbesondere fettigem Haar und/oder Kopfschuppen, **dadurch gekennzeichnet, daß** das oder die Cyclodextrine in einem geeigneten kosmetischen oder dermatologischen Träger, mit dem von gesteigerte Sebumproduktion betroffenen Bereich in Kontakt gebracht werden.

6. Verwendung nach Anspruch 1 oder Verfahren nach Anspruch 5, **dadurch gekennzeichnet, daß** das oder die Cyclodextrine eingesetzt werden, einem Gehalt von 0,0005 - 50,0 Gew.-%, insbesondere 0,01 - 20,0 Gew.-%, entsprechend, bezogen auf das Gesamtgewicht der Zusammensetzung

7. Verwendung nach Anspruch 1 oder Verfahren nach Anspruch 5, **dadurch gekennzeichnet, daß** das oder die Cyclodextrine eingesetzt werden, einem Gehalt von 0,02 - 10,0 Gew.-%, bevorzugt 0,02 - 5,0 Gew.-%, besonders vorteilhaft 0,5 - 3,0 Gew.-%, entsprechend, jeweils bezogen auf das Gesamtgewicht der Zusammensetzung.
